# EUROPEAN PATENT SPECIFICATION

(11) **EP 1 305 020 B1**
(45) Date of publication and mention of the grant of the patent: **21.06.2006**
(21) Application number: 01955528.3
(22) Date of filing: 20.07.2001
(51) Int. Cl.: A61K 31/216, A61K 31/195, A61K 31/192, A61P 9/04

(54) **USE OF FIBRATES FOR THE PREPARATION OF A MEDICAMENT USEFUL IN THE TREATMENT OF CONGESTIVE HEART FAILURE**
VERWENDUNG VON FIBRATE ZUR HERSTELLUNG EINES ARZNEIMITTEL ZUR BEHANDLUNG VON KONGESTIVEN HERZVERSAGENS
UTILISATION DE FIBRATES DANS LA PREPARATION D'UN MEDICAMENT UTILISE DANS LE TRAITEMENT D'INSUFFISANCE CARDIAQUE GLOBALE

(30) Priority: 01.08.2000 IT RM200043
(43) Date of publication of application: 02.05.2003
(73) Proprietor: SIGMA-TAU Industrie Farmaceutiche Riunite S.p.A., 00144 Roma (IT)
(72) Inventor: ARDUINI, Arduino, 00040 Pomezia (IT)
(74) Representative: Spadaro, Marco
(86) International application number: PCT/IT2001/000390
(87) International publication number: WO 2002/009682

(56) References cited:
- WO-A-01/40207
- WO-A-01/57001
- WO-A-98/05331
- WO-A-99/59957
- US-A- 4 925 672
- SCHMIDT-SCHWEDA, S AND HOLUBARSCH, C: "First clinical trial with etomoxir in patients with chronic congestive heart failure" CLINICAL SCIENCE, vol. 99, no. 1, 1 July 2000 (2000-07-01), pages 27-35, XP001027722
- TURCANI, M, RUPP, H: "Etomoxir improves left ventricular performance of pressure-overloaded rat heart" CIRCULATION, vol. 96, no. 10, 18 November 1997 (1997-11-18), pages 3681-3686, XP001027578 cited in the application

## Description

The invention described herein relates to medicaments useful for the treatment of cardiovascular diseases, particularly congestive heart failure, said heart failure being unrelated to dyslipidaemic conditions of any kind.

### Background to the invention

Congestive heart failure (CHF) is an important cause of disability and sudden death (approximately 10%/year), with a high incidence (1-5 cases per 1,000 inhabitants in the younger age) more than 30 cases per 1,000 inhabitants in patients aged above 75 years.

The physiopathological mechanisms involved in the onset, development and progression of CHF have still to be fully clarified. Despite the fact that numerous biochemical, electrophysiological and functional abnormalities have been found, it is hard to establish whether these constitute a cause or a consequence of the disease.

CHF is due to the inability of the heart to pump blood in sufficient amounts to cope with the metabolic needs of the various tissues. This condition is accompanied by profound changes in the control system of the heart's electrical and mechanical functions. The biochemical and neurohormonal abnormalities observed are a mechanism of adaptation to the altered haemodynamic condition of the decompensated heart, characterised mainly by a reduction in cardiac output, an increase in peripheral resistances and retention of blood upstream of the decompensated heart, with consequent atrial dilation and retrograde decompensation.

Etomoxir is an irreversible inhibitor of CPT1, known as a potent serum-glucose-lowering agent both in human subjects and in animal models.

Recently, the possible therapeutic role of inhibition of carnitine palmitoyl transferase 1 (CPT1) in heart failure has been emerging in various experimental models and also in human subjects. The pharmacological action of Etomoxir consists in the inhibition of CPT1, an enzyme located on the inner surface of the external mitochondrial membrane and involved in the transport of long-chain fatty acids into the mitochondria within the framework of the oxidation processes of these acids (McGarry et al. J. Clin. Invest. 52:877-884). By inhibiting the enzyme CPT1, and consequently the oxidation of fatty acids, Etomoxir increases the oxidation of glucose, reducing gluconeogenesis (Selby et al. Trends Pharmacol. Sci. 10:495-500). This phenomenon has been observed in the liver, skeletal muscle and heart with a consequent reduction of β-oxidation and an accumulation of lipids, particularly in the liver and heart (Reinauer et al. J. Clin. Chem. 28:335-339).

Several studies have demonstrated the efficacy of Etomoxir in improving the functional capability of the myocardium in animal models of cardiac hypertrophy and heart failure (Turcani et al. Circulation 1997, 96:3681-3686 and Br. J. Pharmacol 1999, 126:501-507), and a positive effect of Etomoxir treatment has also been observed more recently in human subjects suffering from heart failure (NYHA II-III).

It has recently been demonstrated that, in the heart, Etomoxir and long-chain fatty acids are capable of activating the transcription of the CPT1 gene through PPARα (Brandt et al J. Biol. Chem., 1998, 273: 23786-23792) of which Etomoxir is a known activator.

In international patent application WO 98/05331, filed in the name of Ligand Pharm. Inc, methods are described for the treatment of type II diabetes and cardiovascular diseases associated with the diabetic or prediabetic state. In this reference, a method is described for the treatment of type II diabetes or cardiovascular diseases associated with diabetic or prediabetic conditions, comprising the administration of a combination of two active ingredients with PPARγ and PPARα agonist activity, respectively. The compounds preferably indicated as PPARγ agonists are compounds belonging to the thiazolidinedione class, such as, for example, the glitazones. The compounds preferably indicated as PPARα agonists are compounds belonging to the fibrate class. In one case, a single compound is described endowed with both PPARα and PPARγ agonist activity. The diseases treated are, in addition to type II diabetes, those associated with the complex diabetic picture. Those mentioned are hypertriglyceridaemia, hyperinsulinaemia, hyperfibrinogaemia, hypertension, obesity, and X syndrome. The method described is also effective in HDL cholesterol level elevation, increased insulin sensitivity, the uptake of glucose at adipocyte or muscle cell level, and in the prevention of insulin resistance. As regards the cardiovascular district, the method described applies to the disorders associated with the diabetic or prediabetic condition, and therefore to hypertension, and coronary artery disorders, mainly due to atheromatous phenomena.

### Summary of the invention

It has now surprisingly been found that the fibrates, and particularly clofibrate, which are ligands of PPARα devoid of CPT1 inhibitory activity, are effective in the treatment of congestive heart failure, said heart failure being unrelated to dyslipidaemic conditions of any kind.

One subject of the invention described herein is therefore the use of a ligand of PPARα, the ligand being a fibrate, in the preparation of a medicament useful for the treatment of congestive heart failure.

In an even more preferred embodiment, the use of clofibrate is envisaged in the invention described herein.

Within the framework of the preferred embodiment of the invention described herein, the fibrates as a chemical class are suitable for said embodiment. Examples of fibrates are clofibrate, which is the one preferred according to the invention, gemfibrozil, fenofibrate, bezafibrate, and ciprofibrate. In the context of the invention, what is meant by "fibrates" are the fibrates, their analogues, congeners and derivatives. The fibrates, with the meaning as understood in the invention described herein, can be used as such or in the form of pharmaceutically acceptable derivatives, such as salts, or derivatives that improve the pharmacokinetic aspects, though conserving specific activity (prodrugs).

As regards the industrial aspect of the present invention, the medicaments will be in the form of suitable pharmaceutical formulations (or compositions), prepared according to conventional methods known to the expert in the sector. Examples of pharmaceutical compositions are tablets, capsules, pills, sachets, liquid forms for oral administration, such as solutions, suspensions and emulsions; controlled release forms for oral administration or enteric administration in general; forms for parenteral administration, such as injectable forms.

The following example further illustrates the invention.

### EXAMPLE

### Materials and Methods

Animal-housed male Wistar rats weighing 100-120 g were used: 5 per cage (cage size: 425 mm x 266 mm x 180 mm with a sawdust litter) at a temperature of 21 ±1°C and 50 ±15% humidity with a 12/12 h light/darkness cycle and with 15-20 air changes/hour. The animals were fed with LP ALTROMIN (REIPER) feed and spring water ad libitum

### Induction of cardiac hypertrophy

Left ventricular hypertrophy was induced in rats anaesthetised with Nembutal sodium, by means of constriction of the abdominal aorta with a clip (Ø 0.8 mm) placed in the abdominal aorta between the diaphragm and the renal branches; one group of animals, which was then used as a control group, underwent the same operation but was not submitted to constriction of the aorta (shams).

The animals were thus randomly assigned to the following groups:
Shams: operated on without aortic constriction (n=8)
Controls: operated on with aortic constriction (n=8)
CLO: operated on with aortic constriction and treated for 12 weeks from the day after the operation with clofibrate 0.2% in the feed (60-100 mg/kg/day (n= 11).

### Evaluation of cardiac function

At the end of treatment, cardiac function was evaluated, in the animals anaesthetised with Nembutal sodium, by means of a polyethylene catheter inserted in the left ventricle via the carotid and connected up to a pressure transducer (Statham p23XL) and to an amplifier (Biomedica Mangoni bm 61)

The parameters recorded were: heart rate, ventricular systolic and end-diastolic pressure, and the positive and negative derivatives of ventricular pressure, which were recorded on a personal computer by means of a special data acquisition system (IDAS). The measurements were taken for a period of 30 minutes.

### Macroscopic assessment

At the end of the experiments the animals were sacrificed by means of a lethal dose of Nembutal, the abdominal cavity was opened, the organs were exteriorised in order to verify correct positioning of the aortic clip, and the heart, lungs and liver were then removed and carefully dried and weighed after macroscopic observation of any abnormalities.

### Statistical analysis

The data were expressed as mean ± standard deviation and were compared using Student's *t*-test for independent data. Differences with *P*<0.05 were regarded as statistically significant.

### Results

### Weight parameters

Table 1 presents the weight parameters measured at the end of the experiment; the body weight of the animals does not change significantly either as a result of constriction of the aorta or as a result of the treatments. Aortic constriction induces a significant ventricular hypertrophy; in fact, the heart weight of the animals with the clip increases by approximately 35% as compared to the shams (P<0.05) (Table 1); the treatments administered do not modify heart weight as compared to the untreated controls. Liver and lung weights were not changed either as a result of aortic constriction or as a result of the treatments administered.

**TABLE 1 Body weight and ventricular, lung and liver weight (BW = body weight (g); VW = ventricular weight (mg), LW = lung weight (mg), LvW = liver weight (mg))**

| | Shams | Controls | CLO |
|---|---|---|---|
| BW | 396±11 | 376±9 | 392 ± 12 |
| VW/BW | 2.42±0.04 | 3.23±0.08**a** | 3.33±0.20**a** |
| L W/BW | 3.79±0.17 | 3.61±0.10 | 3.49±0.07 |
| LvW/BW | 28.9±1.2 | 27.0±1.5 | 29.2±1.8 |

| | | | |
|---|---|---|---|
| **a** = *P*<0.05 vs shams | | | |

### Cardiac function

Aortic constriction induces a significant increase both in left ventricular systolic pressure and in end-diastolic pressure; the pressure developed, and the positive and negative derivatives of ventricular pressure show no statistically significant changes in absolute values in the animals with aortic constriction as compared to the shams (Table 2).

If the pressure developed is normalised for ventricular weight, a statistically significant reduction in this parameter is observed in the animals with aortic constriction; similarly, the positive derivative of ventricular pressure is significantly reduced if it is normalised for ventricular systolic pressure (Table 2).

Treatment of the animals with aortic constriction normalises end-diastolic pressure, and the pressure developed in relation to ventricular weight, with a significant improvement in both the positive and negative derivatives of ventricular pressure (Table 2).

Treatment with clofibrate induces a further increase in left ventricular systolic pressure and normalises end-diastolic pressure in the animals with aortic constriction.

The pressure developed in relation to ventricular weight, and both the positive and negative derivatives of ventricular pressure are significantly improved by treatment with clofibrate (Table 2).
The results are summarised in Table 2, in which HR = heart rate (b/min); LVSP = left ventricular systolic pressure (mm Hg); LVEDP = left ventricular end-diastolic pressure LdevP developed pressure (mm Hg); HW = ventricular weight (mg), DP/dt max = positive derivative of ventricular pressure (mm Hg/sec); DP/dt min = negative derivative of ventricular pressure (mm Hg/sec).

**Table 2 Cardiac functional parameters**

| | | | |
|---|---|---|---|
| | Sham | Controls | CLO |
| HR | 357±18 | 382±13 | 401±16 |
| LVSP | 147±5 | 182±11**a** | 206±5**aB** |
| LVEDP | 2.5±2.1 | 16.7±4.2**b** | 7.2±1.2**A** |
| LdevP | 150±5 | 169±15 | 207±7**cA** |
| LDevP/HW | 7.9±0.9 | 5.4±0.5**a** | 13.3±1.5**aC** |
| DP/dt max | 7434±559 | 6709±1080 | 16108±1115e C |
| DP/dt min | 4849±492 | 5342±993 | 9556±560**bA** |
| DP/dtmax/LVSP | 51±4 | 36±5**a** | 77±5**eC** |

| | | | |
|---|---|---|---|
| a= *P*<0.05; b= *P*<0.01; c= *P*<0.001 vs shams; A= *P*<0.05; B= *P*<0.01; C= *P*<0.001 vs Controls | | | |

## Claims

1. Use of a ligand of PPARα, the ligand being a fibrate, for the preparation of a medicament useful in the treatment of congestive heart failure, said heart failure being unrelated to dyslipidaemic conditions of any kind.

2. Use according to claim 1, where said fibrate is selected from the group consisting of gemfibrozil, fenofibrate, bezafibrate, ciprofibrate and clofibrate.

3. Use according to claim 2, where said fibrate is clofibrate.

## Patentansprüche

1. Verwendung eines Liganden von PPARα, wobei der Ligand ein Fibrat ist, für die Herstellung eines Medikaments, das für die Behandlung von kongestivem Herzversagen nützlich ist, wobei das Herzversagen mit dyslipidämischen Zuständen irgendeiner Art nicht in Beziehung steht.

2. Verwendung gemäß Anspruch 1, wobei das Fibrat gewählt ist aus der Gruppe bestehend aus Gemfibrozil, Fenofibrat, Bezafibrat, Ciprofibrat und Clofibrat.

3. Verwendung gemäß Anspruch 2, wobei das Fibrat Clofibrat ist.

## Revendications

1. Utilisation d'un ligand de PPARα, ledit ligand étant un fibrate, pour la préparation d'un médicament destiné au traitement de l'insuffisance cardiaque congestive, ladite insuffisance cardiaque étant sans relation avec des états dyslipidémiques de type quelconque.

2. Utilisation selon la revendication 1, dans laquelle ledit fibrate est choisi dans le groupe constitué du gemfibrozil, du fénofibrate, du bézafibrate, du ciprofibrate et du clofibrate.

3. Utilisation selon la revendication 2, dans laquelle ledit fibrate est le clofibrate.
